# EUROPÄISCHE PATENTANMELDUNG

(11) **EP 4 656 825 A1**
(43) Veröffentlichungstag der Anmeldung: **03.12.2025**
(21) Anmeldenummer: 25177196.0
(22) Anmeldetag: 19.05.2025
(51) Int. Cl.: E05B 65/00, G08B 17/113, A62C 13/78, E05B 47/00, A62C 35/00

(54) **ANORDNUNG MIT EINEM GASMESSGERÄT UND EINEM BEHÄLTER UND VERFAHREN UNTER VERWENDUNG EINER SOLCHEN ANORDNUNG**

(30) Priorität: 30.05.2024 DE 102024115154
(71) Anmelder: Dräger Safety AG & Co. KGaA, 23560 Lübeck (DE)
(72) Erfinder: Sturm, Hannes, 23558 Lübeck (DE); Hansen, Florian, 23558 Lübeck (DE); Wilhelm, Christian, 23558 Lübeck (DE)

(57) **Zusammenfassung**

Die Erfindung betrifft eine Anordnung und ein Verfahren, welche in einen Bereich, in dem sich Menschen aufhalten oder aufhalten können, überwachen. Ein Gasmessgerät (1.1, 1.2) misst in seinem Detektionsbereich (Det.1, Det.2) die Konzentration eines Zielgases und generiert ein Signal, welches eine Information über die gemessene Zielgas-Konzentration umfasst. Ein Behälter (5) nimmt in seinem Innenraum einen Gegenstand (6, 7, 8, 9, 14) auf, insbesondere zum Retten von Menschen. Ein Verschließmechanismus (4) verschließt in einem verschließenden Zustand den Behälter (5), so dass ein Zugriff auf den Gegenstand im Behälter (5) versperrt ist. In einem freigebenden Zustand ermöglicht der Verschließmechanismus (4), dass der Gegenstand aus dem Behälter entnommen wird. Ein signalverarbeitendes Steuergerät (10) empfängt und verarbeitet das Signal vom Gasmessgerät (1.1, 1.2) und entscheidet automatisch, ob eine zu hohe Zielgas-Konzentration vorliegt. Bei einer zu hohen Zielgas-Konzentration bewirkt das Steuergerät (10), dass der Verschließmechanismus (4) in den freigebenden Zustand überführt wird.

## Beschreibung

Die Erfindung betrifft eine Anordnung und ein Verfahren, welche einen räumlichen Bereich, in dem sich Menschen aufhalten oder aufhalten können, auf gefährliche Situationen zu überwachen vermögen. Der Bereich befindet sich insbesondere im Inneren eines Gebäudes oder eines Fahrzeugs, kann aber auch ein Bereich im Freien sein.

Der Erfindung liegt die Aufgabe zugrunde, eine Anordnung und ein Verfahren bereitzustellen, welche besser als bekannte Anordnungen ermöglichen, den Schutz von Menschen in einem räumlichen Bereich zu ermöglichen, wobei diese Menschen durch zu viel schädliches Gas oder auch zu wenig lebensnotwendiges Gas gefährdet werden können.

Die Aufgabe wird durch eine Anordnung mit den Merkmalen des Anspruchs 1 und durch ein Verfahren mit den Merkmalen des Anspruchs 8 gelöst. Vorteilhafte Ausgestaltungen sind in den Unteransprüchen angegeben. Vorteilhafte Ausgestaltungen der erfindungsgemäßen Anordnung sind, soweit sinnvoll, auch vorteilhafte Ausgestaltungen des erfindungsgemäßen Verfahrens und umgekehrt.

Die erfindungsgemäße Anordnung umfasst mindestens ein Gasmessgerät, optional mehrere Gasmessgeräte. Das oder jedes Gasmessgerät der Anordnung ist dazu ausgestaltet, an jeweils einem Ort aufgestellt zu werden, und weist jeweils einen räumlichen Detektionsbereich auf. In einer Ausgestaltung lässt sich das oder ein Gasmessgerät an ein stationäres Spannungsversorgungsnetz anschließen. In einer Ausgestaltung umfasst das oder ein Gasmessgerät eine eigene Spannungsversorgungseinheit, um unabhängig von einem stationären Spannungsversorgungsnetz zu sein. Diese beiden Ausgestaltungen lassen sich miteinander kombinieren.

Das oder jedes Gasmessgerät ist dazu ausgestaltet, die Konzentration jeweils mindestens eines Zielgases in seinem jeweiligen Detektionsbereich zu messen. Ein Gas, dessen Konzentration von dem oder mindestens einem Gasmessgerät der Anordnung gemessen werden kann, wird nachfolgend als ein "Zielgas" bezeichnet. In einer Ausgestaltung vermag das Gasmessgerät die Konzentration eines Zielgases zu messen, in einer anderen Ausgestaltung die jeweilige Konzentration von mehreren Zielgasen oder die Summe der Zielgas-Konzentrationen. In einer Realisierungsform vermag das Gasmessgerät die jeweilige Konzentration von mehreren gleichzeitig auftretenden Zielgas zu messen, in einer anderen Realisierungsform lässt sich das Gasmessgerät beispielsweise mithilfe eines Schalters konfigurieren oder einstellen, wobei es von der Konfiguration oder Einstellung abhängt, von welchem Zielgas das Gasmessgerät die Konzentration misst. Es ist möglich, dass zwei verschiedene Gasmessgeräte der Anordnung die Konzentration desselben Zielgas zu messen vermögen.

Der Detektionsbereich des Gasmessgeräts oder die Detektionsbereiche der Gasmessgeräte der Anordnung zusammen deckt / decken idealerweise einen räumlichen Bereich ab, in dem sich Menschen aufhalten oder aufhalten können und in dem mindestens ein Zielgas auftritt oder auftreten kann.

Das oder mindestens ein Zielgas kann ein Gas sein, das in einer ausreichend hohen Konzentration für Menschen gefährlich ist, insbesondere ein brennbares oder giftiges Zielgas. Das oder ein Zielgas kann auch für Menschen lebensnotwendig sein, beispielsweise Sauerstoff, oder ein Narkosemittel. In vielen Fällen vermag ein Mensch mit keinem seiner fünf Sinne festzustellen, dass die Zielgas-Konzentration außerhalb eines vorgegebenen Wertebereichs liegt. Daher ist oft ein Gasmessgerät erforderlich, um diese gefährliche Situation zu detektieren.

Anmerkung: Die Formulierung wird verwendet, dass ein Sensor eine physikalische Größe zu messen vermag, beispielsweise die Konzentration eines Zielgases. Diese Formulierung bedeutet, dass der Sensor direkt die physikalische Größe zu messen vermag oder mindestens eine andere Größe, die mit der zu messenden Größe korreliert. Die oder eine gemessene andere Größe oder die Kombination der gemessenen anderen Größen zusammen sind daher ein Maß für die zu messende physikalische Größe. Die Messung liefert mindestens einen Wert für die gesuchte physikalische Größe.

Weiterhin vermag das oder jedes Gasmessgerät jeweils ein Signal zu generieren. Dieses Signal umfasst eine Information über die oder jede vom generierenden Gasmessgerät gemessene Zielgas-Konzentration.

Weiterhin umfasst die Anordnung einen Behälter. Dieser Behälter umfasst eine Wand, die einen Innenraum umschließt, wobei bevorzugt in die Wand eine Tür oder ein Deckel oder ein sonstiger Verschluss eingelassen ist. Der Behälter ist dazu ausgestaltet, in seinem Innenraum mindestens einen Gegenstand aufzunehmen. Bevorzugt ist der oder mindestens ein im Innenraum aufgenommener Gegenstand einer der folgenden Gegenstände:
- eine persönliche Schutzausrüstung oder ein Bestandteil einer persönlichen Schutzausrüstung, beispielsweise ein Schutzhelm oder ein Selbstretter mit einem Generator für ein atembares Gasgemisch und einer Atemmaske,
- ein Werkzeug, mit dem ein Mensch einen Brand bekämpfen kann, beispielsweise ein Feuerlöscher oder eine Feuerlöschdecke,
- ein Werkzeug, mit dem ein Mensch sich Zugang zu einem Raum verschaffen oder einen solchen Zugang vergrößern kann, beispielsweise ein Aufbrechwerkzeug oder eine Ramme,
- ein Werkzeug, mit dem ein Mensch ein Leck in einer Leitung abdichten kann,
- eine tragbare Lichtquelle, insbesondere eine Taschenlampe,
- eine tragbare Alarmeinheit, beispielsweise eine Meldeleuchte oder eine Hupe oder eine Sirene,
- ein tragbares Gasmessgerät, welches die Konzentration eines Zielgases zu messen vermag und bevorzugt eine eigene Ausgabeeinheit und eine eigene Spannungsversorgungseinheit aufweist,
- ein tragbarer Rauchwarnmelder.

In der Regel ist in die Wand des Behälters eine Öffnung eingelassen, und ein beweglicher Verschluss, beispielsweise eine Tür oder ein Deckel, verschließt je nach Stellung dieser Öffnung oder gibt diese Öffnung frei.

Ein ansteuerbarer Verschließmechanismus der Anordnung lässt sich wahlweise in einen verschließenden oder in einen freigebenden Zustand überführen. Im verschließenden Zustand verschließt der Verschließmechanismus den Behälter derart, dass ein Zugriff auf den Innenraum und damit auf den oder jeden im Behälter - genauer gesagt: im Innenraum des Behälters - befindlichen Gegenstand versperrt ist. In der Regel verschließt der Verschließmechanismus im verschließenden Zustand den Verschluss und verhindert dadurch, dass der Verschluss sich bewegen lässt und dadurch die Öffnung in der Wand des Behälters freigibt. Im freigebenden Zustand ermöglicht der Verschließmechanismus es, dass sich mindestens ein, bevorzugt jeder Gegenstand aus dem Inneren des Behälters entnehmen lässt. In der Regel lässt sich dann der Verschluss bewegen und dadurch öffnen, und dadurch ist ein Zugriff durch die Öffnung in der Wand möglich.

Ein signalverarbeitendes Steuergerät (control unit) der Anordnung steht dauerhaft oder wenigstens zeitweise in jeweils einer Datenverbindung mit dem oder jedem Gasmessgerät der Anordnung und bevorzugt in jeweils einer Datenverbindung mit mindestens einem, bevorzugt jedem optionalen weiteren Sensor der Anordnung, beispielsweise einem Rauchwarnmelder. Die oder jede Datenverbindung kann mittels einer Datenleitung und / oder drahtlos, also mittels Funkwellen, hergestellt sein. Über diese Datenverbindung vermag das Steuergerät von jedem Gasmessgerät der Anordnung dasjenige Signal zu empfangen, welches dieses Gasmessgerät erzeugt hat und welches eine Information über eine von diesem Gasmessgerät gemessene Zielgas-Konzentration umfasst. Bevorzugt fragt das Steuergerät wiederholt das oder jedes Gasmessgerät ab und überprüft auf diese Weise, ob das Gasmessgerät noch aktiv ist.

Das Steuergerät vermag das oder jedes empfangene Signal zu verarbeiten. Das Steuergerät vermag folgendes zu entscheiden: Tritt im Detektionsbereich des oder mindestens eines Gasmessgeräts der Anordnung ein Zielgas mit einer Konzentration außerhalb eines vorgegebenen Wertebereichs, insbesondere oberhalb einer vorgegebenen oberen Schranke, auf oder nicht? Eine Konzentration außerhalb des Wertebereichs wird auch als schädliche oder unzulässige Zielgas-Konzentration bezeichnet. Diese Entscheidung fällt das Steuergerät automatisch und in Abhängigkeit von mindestens einem verarbeiteten Signal eines Gasmessgeräts der Anordnung. Falls das Zielgas für Menschen schädlich sein kann, so ist in der Regel als Wertebereich eine obere Schranke vorgegeben, und zwar dergestalt, dass eine Konzentration dieses Zielgases unterhalb der oberen Schranke für Menschen nicht gefährlich ist. Entsprechend ist in der Regel für ein lebensnotwendiges Gas, insbesondere Sauerstoff, als Wertebereich eine untere Schranke vorgegeben. Der vorgegebene Wertebereich kann von Zielgas zu Zielgas differieren.

Solange die oder jede Zielgas-Konzentration im jeweils vorgegebenen Wertebereich, insbesondere unterhalb der jeweiligen oberen Schranke, liegt und auch eine sonstige für Menschen gefährliche Situation nicht detektiert ist, ist der Verschließmechanismus im verschließenden Zustand. Zu Beginn des erfindungsgemäßen Verfahrens ist der Verschließmechanismus im verschließenden Zustand. Falls das Steuergerät detektiert hat, dass in dem oder mindestens einem Detektionsbereich eine schädliche Zielgas-Konzentration auftritt, so bewirkt das Steuergerät folgendes: Das Steuergerät steuert den Verschließmechanismus an. Durch die Ansteuerung wird der Verschließmechanismus in den freigebenden Zustand überführt. Dadurch lässt der Behälter sich öffnen oder wird automatisch geöffnet. Beispielsweise springt eine Tür des Behälters dank eines Federmechanismus auf. Nunmehr ist es möglich, den oder mindestens einen Gegenstand aus dem Behälter zu entnehmen und zu nutzen.

Das erfindungsgemäße Verfahren umfasst die entsprechenden Schritte.

Falls die Konzentration des oder eines Zielgases außerhalb des vorgegebenen Wertebereichs liegt, kann eine Situation auftreten, die für einen Menschen gefährlich ist, falls der Mensch sich im überwachten räumlichen Bereich und daher im Detektionsbereich des oder mindestens eines Gasmessgeräts der Anordnung aufhält. Die Erfindung erleichtert es, dass ein Mensch sich vor einer schädlichen Zielgas-Konzentration schützt und / oder ein Austreten eines schädlichen Zielgases reduziert oder wenigstens detektiert wird. Entsprechend erleichtert die Erfindung es, dass ein Mensch sich vor den Auswirkungen einer zu geringen Konzentration eines lebensnotwendigen Zielgases schützt.

Mindestens ein hierfür geeigneter Gegenstand ist in dem Behälter vorhanden und lässt sich aus dem Behälter entnehmen, wenn der Verschließmechanismus im freigebenden Zustand ist.

Möglich wäre es, überhaupt keinen Behälter für den Gegenstand vorzusehen oder den Behälter so auszugestalten, dass sich jederzeit aus dem Behälter mindestens ein Gegenstand entnehmen lässt. Dann aber bestünde die Gefahr, dass der Gegenstand unberechtigterweise aus dem Behälter entnommen wird und dann der Gegenstand nicht mehr vorhanden ist, wenn er benötigt wird. Dieses Problem ist beispielsweise von öffentlich zugänglichen Rettungsringen bekannt. Der erfindungsgemäße Verschließmechanismus verhindert völlig oder reduziert zumindest die Gefahr, dass ein Gegenstand unberechtigterweise, nämlich außerhalb einer Notsituation, aus dem Behälter entnommen wird und dann im Notfall fehlt. Außerdem reduziert der Verschließmechanismus die Gefahr, dass ein Gegenstand außerhalb einer Notsituation entnommen und benutzt und dann wieder zurück gelegt wird und dann im Notfall seine lebensrettende Funktion überhaupt nicht oder nicht ausreichend sicher erfüllen kann.

Möglich ist, dass der erfindungsgemäße Verschließmechanismus
- ein mechanisches Schloss, das sich mit einem passenden mechanischen Schlüssel öffnen lässt, und / oder
- einen Chipkartenleser für eine passende Chipkarte und / oder
- eine Eingabeeinheit für ein Passwort und / oder
- ein Lesegerät für ein biometrisches Merkmal einer Person.
umfasst. Diese Ausgestaltung ermöglicht es, dass eine berechtigte Person einen Gegenstand im Behälter benutzt oder überprüft oder austauscht, und zwar auch außerhalb einer Notsituation, wenn kein Zeitdruck besteht.

Die Gefahr ist aber groß, dass in einer Notsituation ein passender Schlüssel oder eine passende Chipkarte überhaupt nicht oder erst nach einer längeren Suchzeit gefunden wird. In einer Notsituation könnte das Passwort nicht verfügbar sein. Außerdem besteht die Gefahr, dass in einer Notsituation ein Mensch in Panik gerät und es ihm nicht gelingt, den richtigen Schlüssel zu finden, das richtige Passwort einzugeben oder die Chipkarte richtig zu verwenden. Ein Mensch, der ein Passwort kennt oder von dem ein biometrisches Merkmal abgespeichert ist, kann in einer Notsituation nicht vor Ort sein.

Das Steuergerät entscheidet automatisch, ob mindestens ein Zielgas mit einer Konzentration außerhalb des jeweils vorgegebenen Wertebereichs vorliegt oder nicht. Als Reaktion darauf, dass eine Konzentration außerhalb des Wertebereichs vorliegt, steuert das Steuergerät den Verschließmechanismus an und bewirkt dadurch, dass der Verschließmechanismus in den freigebenden Zustand überführt wird und sich mindestens ein Gegenstand aus dem Behälter entnehmen lässt. Die Erfindung erspart insbesondere dank dieses Merkmals die Notwendigkeit, dass ein Mensch mit Hilfe eines Schlüssels oder einer Chipkarte oder eines Passworts oder eines biometrischen Merkmals den Behälter öffnen muss. Vielmehr steuert erfindungsgemäß das Steuergerät bei einer schädlichen Zielgas-Konzentration den Verschließmechanismus an und bewirkt dadurch, dass der Verschließmechanismus in den freigebenden Zustand überführt wird und sich nunmehr ein Gegenstand aus dem Behälter entnehmen lässt. In einer Notsituation werden weder ein mechanischer Schlüssel noch eine Chipkarte noch ein Passwort noch ein biometrisches Merkmal benötigt.

Die erfindungsgemäße Anordnung lässt sich in Kombination mit einer Alarmeinheit einsetzen, wobei diese Alarmeinheit in mindestens einer von einem Menschen wahrnehmbaren Form einen Alarm ausgibt, wenn eine Zielgas-Konzentration außerhalb des Wertebereichs detektiert worden ist, insbesondere visuell und / oder akustisch. Die Anordnung lässt sich auch in Kombination mit einer Notfallzentrale verwenden, wobei die Notfallzentrale Signale der Gasmessgeräte und optional Signale weiterer Sensoren empfängt und anzeigt und wobei ein Mensch in der Notfallzentrale aus der Ferne bei einer schädlichen Zielgas-Konzentration mindestens eine geeignete Rettungsmaßnahme auslöst und insbesondere bewirkt, dass der Behälter geöffnet wird.

Jedoch erspart die Erfindung die Notwendigkeit, dass ein Mensch vor Ort oder in einer Notfallzentrale die Ausgabe eines Alarms auslöst oder wahrnimmt und als Reaktion auf den Alarm den Behälter öffnet oder bewirkt, dass der Behälter geöffnet wird. Vielmehr bewirkt dies das Steuergerät automatisch. Dieses Merkmal der erfindungsgemäßen Anordnung reduziert die Gefahr, dass ein Mensch im Gefahrenbereich aufgrund einer eigenen Fehlentscheidung oder aufgrund einer falschen oder fehlenden Entscheidung eines anderen Menschen gefährdet wird. Die Fehlentscheidung kann insbesondere daraus resultieren, dass ein Mensch einen Alarm nicht wahrnimmt. Außerdem spart die Erfindung in vielen Fällen Zeit ein, insbesondere weil ein Steuergerät oft schneller reagiert als ein Mensch.

Erfindungsgemäß misst das oder jedes Gasmessgerät der Anordnung jeweils eine Zielgas-Konzentration. Bevorzugt entscheidet das Steuergerät automatisch, ob die oder jede gemessene Zielgas-Konzentration in dem oder außerhalb des jeweiligen Wertebereichs liegt. Dieses Merkmal erspart die Notwendigkeit, eine rechnerauswertbare Kennzeichnung des jeweiligen Wertebereichs in dem oder jedem Gasmessgerät der Anordnung selbst abzuspeichern und bei Bedarf zu verändern. Vielmehr reicht es aus, die Kennzeichnung der Wertebereiche im Steuergerät abzuspeichern. Um zu entscheiden, ob eine Notsituation vorliegt, kann das Steuergerät auch eine vorgegebene Auswertevorschrift anwenden, wobei diese Auswertevorschrift von mindestens einer Zielgas-Konzentration und optional von mindestens einem sonstigen Messwert, beispielsweise von einer weiteren gemessenen Zielgas-Konzentration oder einer gemessenen Umgebungsbedingung oder dem Auftreten von Rauch, abhängt. Die Auswertungsvorschrift kann also von den Signalen mehrerer Sensoren abhängen.

Wie bereits dargelegt, überdecken die Detektionsbereiche der Gasmessgeräte der Anordnung zusammen einen zu überwachenden räumlichen Bereich, wobei sich in diesem räumlichen Bereich Menschen aufhalten oder wenigstens aufhalten können und wobei in diesem räumlichen Bereich eine unzulässige Zielgas-Konzentration auftreten kann. Bevorzugt befindet der Behälter sich in diesem überwachten räumlichen Bereich oder wenigstens in der Nähe des räumlichen Bereichs, sodass ein Mensch, der sich im räumlichen Bereich aufhält, bei Bedarf rasch den Behälter erreichen und aus dem Behälter einen Gegenstand entnehmen kann. Das Steuergerät kann sich hingegen außerhalb des räumlichen Bereichs befinden. Dies erleichtert es, das Steuergerät vor schädlichen Einwirkungen zu schützen, die im räumlichen Bereich auftreten können. Weiterhin erleichtert es diese Ausgestaltung, sicherzustellen, dass das Steuergerät stets mit elektrischer Energie versorgt und eine Störung des Steuergeräts rasch erkannt wird.

Erfindungsgemäß verschließt der Verschließmechanismus im verschließenden Zustand den Behälter. Im freigebenden Zustand wird ermöglicht, dass sich ein Gegenstand aus dem Behälter entnehmen lässt. In einer Ausgestaltung umfasst der Verschließmechanismus einen Arretierkörper, ein Rückstellelement und ein Stellglied. Der Arretierkörper ist relativ zur Wand des Behälters und damit relativ zu einem Verschluss, insbesondere zu einer Tür, des Behälters zwischen einer verschließenden und einer freigebenden Position hin und her beweglich. Wenn der Arretierkörper in der verschließenden Position ist, ist der Verschließmechanismus im verschließenden Zustand. Wenn der Arretierkörper in der freigebenden Position ist, ist der Verschließmechanismus im freigebenden Zustand.

Das Rückstellelement übt eine rückstellende Kraft aus und ist bestrebt, mittels der rückstellenden Kraft den Arretierkörper in die eine der beiden Positionen und damit den Verschließmechanismus in den einen der beiden Zustände zu überführen und in diesem zu halten. Das Stellglied lässt sich durch eine Ansteuerung von außen aktivieren und deaktivieren. Bei deaktiviertem Stellglied wird nur das Rückstellelement auf den Arretierkörper. Das aktivierte Stellglied ist bestrebt, gegen die rückstellende Kraft den Arretierkörper in die andere der beiden Positionen und damit den Verschließmechanismus in den anderen der beiden Zustände zu überführen. Das Steuergerät vermag das Stellglied anzusteuern. Eine Ansteuerung bewirkt, dass das Stellglied aktiviert oder deaktiviert wird. Das Rückstellelement ist bevorzugt als ein passives Bauteil ausgestaltet, welches keine Versorgung mit elektrischer oder hydraulischer oder pneumatischer Energie benötigt und auch nicht angesteuert zu werden braucht.

Möglich ist, dass das Rückstellelement bestrebt ist, den Arretierkörper in die verschließende Position zu bewegen und dadurch den Verschließmechanismus im verschließenden Zustand zu halten. Bevorzugt ist hingegen das Rückstellelement bestrebt, den Arretierkörper in die freigebende Position zu bewegen und dadurch den Verschließmechanismus in den freigebenden Zustand zu überführen. Das aktivierte Stellglied hält den Verschließmechanismus gegen die rückstellende Kraft im verschließenden Zustand. Falls das Steuergerät eine schädliche Zielgas-Konzentration detektiert hat, so bewirkt das Steuergerät durch eine entsprechende Anordnung, dass das Stellglied deaktiviert wird. Dann überführt das Rückstellelement mittels der rückstellenden Kraft den Arretierkörper in die freigebende Position.

Ein Hintergrund dieser bevorzugten Realisierungsform ist der folgende: In der Regel benötigt das Stellglied elektrische Energie, um die rückstellende Kraft zu überwinden. Falls das Stellglied oder die Versorgung mit elektrischer Energie ausfallen, vermag das Stellglied diese Wirkung nicht mehr auszuführen. Ein sicherer Zustand ist hergestellt, wenn bei einem Ausfall der Energieversorgung der Verschließmechanismus in den freigebenden Zustand überführt wird. Gerade bei einer schädlichen Zielgas-Konzentration, optional in Verbindung mit einem Feuer, wird manchmal die Energieversorgung gewollt abgeschaltet oder fällt aus.

In einer Ausgestaltung kann ein Mensch einen Verschluss des Behälters öffnen, beispielsweise eine Tür verschwenken, wenn der Verschließmechanismus im freigebenden Zustand ist. In einer bevorzugten Ausgestaltung umfasst der Behälter einen Öffnungsmechanismus. Bevorzugt ist auch der Öffnungsmechanismus als passives Bauteil ausgeführt und benötigt keine elektrische oder pneumatische oder hydraulische Energie und keine Ansteuerung. Der Öffnungsmechanismus ist bestrebt, den Verschluss zu öffnen, so dass der Verschluss aufspringt, wenn der Verschließmechanismus in den freigebenden Zustand überführt wird, ohne dass eine Handlung eines Menschen erforderlich ist, um den Verschluss zu öffnen. In einer Realisierungsform umfasst der Öffnungsmechanismus eine mechanische oder pneumatische Feder.

In einer Ausgestaltung umfasst die Anordnung zusätzlich einen Rauchwarnmelder. Dieser Rauchwarnmelder vermag in seinem Detektionsbereich Rauch zu detektieren. Bekanntlich sind Rauch in einer höheren Konzentration und Feuer ebenfalls schädlich für Menschen, nämlich weil Menschen eine Rauchvergiftung oder auch Verbrennungen erleiden können. Falls der Rauchwarnmelder Rauch detektiert hat, so generiert der Rauchwarnmelder ein Signal, wobei das Signal eine Information über die Detektion von Rauch umfasst. Dieses Signal wird an das Steuergerät übermittelt.

Als Reaktion auf den Empfang dieses Signals bewirkt das Steuergerät, dass der Verschließmechanismus in den freigebenden Zustand überführt wird, und zwar bevorzugt unabhängig von einer gemessenen Zielgas-Konzentration.

Diese Ausgestaltung führt dazu, dass sowohl im Falle einer unzulässigen Zielgas-Konzentration als auch im Falle eines Brandes oder von Rauch der Behälter geöffnet wird und sich ein Gegenstand aus dem Behälter entnehmen lässt. Optional gibt der Rauchwarnmelder zusätzlich einen Alarm aus, bevorzugt in akustischer Form. Möglich, aber dank dieser Ausgestaltung nicht erforderlich ist, dass ein Mensch den Alarm wahrnimmt und dann richtig auf den Alarm reagiert.

In einer Ausgestaltung umfasst die Anordnung mindestens eine Meldeeinheit. In eine Realisierungsform ist die oder eine Meldeeinheit am Behälter angeordnet, oder in der Nähe des Behälters ist mindestens eine Meldeeinheit angeordnet. In einer anderen Realisierungsform ist die oder eine Meldeeinheit ein tragbares Gerät und lässt sich von einem Menschen mit sich führen. Möglich ist, dass die Anordnung mindestens eine Meldeeinheit am Behälter und zusätzlich mindestens eine tragbare Meldeeinheit umfasst. Die oder jede Meldeeinheit lässt sich durch eine Ansteuerung von außen automatisch aktivieren. Als Reaktion darauf, aktiviert worden zu sein, tätigt die Meldeeinheit eine Ausgabe in mindestens einer von einem Menschen wahrnehmbaren Form, insbesondere visuell oder akustisch. Ein Mensch kann diese Ausgabe von außerhalb des Behälters wahrnehmen, also auch dann, wenn der Behälter verschlossen ist. Beispielsweise umfasst die Meldeeinheit eine Meldeleuchte oder eine Hupe oder eine Sirene. Möglich ist, dass die Anordnung eine erste Meldeeinheit für eine visuelle Ausgabe und eine zweite Meldeeinheit für eine akustische Ausgabe umfasst. Die oder eine Meldeeinheit kann auch innerhalb des Behälters angeordnet sein, vorausgesetzt eine Ausgabe der Meldeeinheit lässt sich auch bei verschlossenen Behälter von außerhalb des Behälters wahrnehmen. Dies gilt insbesondere für eine ausreichend laute akustische Ausgabe.

Solange die oder jede Zielgas-Konzentration im jeweiligen Wertebereich liegt und der optionale Rauchwarnmelder keinen Rauch detektiert hat, ist die Meldeeinheit nicht aktiviert und tätigt keine Ausgabe oder eine Standard-ausgabe.

Erfindungsgemäß überführt das Steuergerät den Verschließmechanismus in den freigebenden Zustand, wenn eine schädliche Zielgas-Konzentration vorliegt oder wenn der optionale Rauchwarnmelder Rauch detektiert hat. Gemäß der Ausgestaltung mit der Meldeeinheit steuert in dieser Situation das Steuergerät zusätzlich die Meldeeinheit an und aktiviert dadurch die Meldeeinheit. Die aktivierte Meldeeinheit tätigt die oben beschriebene Ausgabe. In einer Ausgestaltung tätigt die Meldeeinheit nach der Aktivierung die Ausgabe auf eine andere Weise als vor einer Aktivierung.

Die Meldeeinheit erleichtert es in vielen Fällen einem Menschen, den Behälter rasch zu finden, insbesondere wenn die Meldeeinheit auf dem Behälter montiert ist. Weil die Meldeeinheit die Ausgabe nicht dauerhaft in der gleichen Weise tätigt, sondern überhaupt erst oder anders nach einer Aktivierung durch eine Ansteuerung, fällt die Ausgabe der Meldeeinheit mehr auf, als wenn die Meldeeinheit dauerhaft die gleiche Ausgabe tätigen würde.

In einer Ausgestaltung umfasst die Anordnung zusätzlich eine Anzeigeeinheit, die sich von außen ansteuern lässt. Als Reaktion auf eine Ansteuerung gibt die Anzeigeeinheit mindestens eine Information in mindestens einer von einem Menschen wahrnehmbaren Weise aus, insbesondere visuell. Diese Information lässt sich von außerhalb des Behälters wahrnehmen und umfasst insbesondere eine der folgenden Informationen:
- einen akustisch und / oder visuell wahrnehmbaren Alarm, dass eine zu hohe oder zu niedrige Zielgas-Konzentration und / oder optional Rauch aufgetreten ist,
- eine Information über einen Weg zu dem Behälter mit dem Gegenstand und dem Verschließmechanismus, beispielsweise eine Richtungsanzeige,
- eine Information über eine räumliche Position des Behälters mit dem Gegenstand und dem Verschließmechanismus, beispielsweise gemäß einer vorgegebenen Kodierung für Bereiche in einem Gebäude.

Optional vermag die angesteuerte Anzeigeeinheit zusätzlich eine Kennzeichnung dafür auszugeben, wie gravierend und / oder wie gefährlich die aktuelle Situation ist, und / oder eine Aufforderung, nunmehr den räumlichen Bereich zu verlassen.

Erfindungsgemäß vermag das Steuergerät den Verschließmechanismus anzusteuern. In einer Ausgestaltung vermag das Steuergerät zusätzlich die gerade beschriebene Anzeigeeinheit anzusteuern. Das Steuergerät steuert die Anzeigeeinheit an, falls eine schädliche Zielgas-Konzentration gemessen und / oder falls der optionale Rauchwarnmelder Rauch detektiert hat.

Die Anzeigeeinheit erleichtert es einem Menschen, rasch den Behälter mit dem Verschließmechanismus zu finden, insbesondere dann, wenn der Mensch sich in einem Gebäude mit dem Behälter nicht auskennt.

Im Folgenden wird die Erfindung anhand eines Ausführungsbeispiels beschrieben. Hierbei zeigt
- Figur 1: die Bestandteile der Anordnung;
- Figur 2: eine Ausgestaltung des Verschließmechanismus.

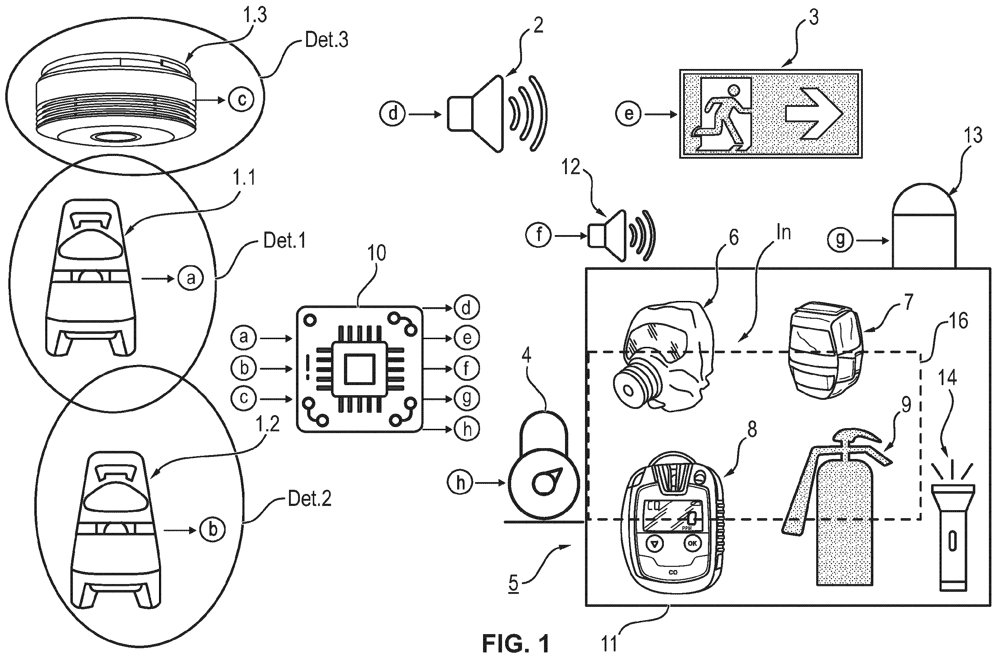

Figur 1 zeigt eine beispielhafte Ausgestaltung der erfindungsgemäßen Anordnung. Die Bestandteile dieser Anordnung werden nachfolgend erläutert.

Die Anordnung wird in einem räumlichen Bereich eingesetzt, in dem sich Menschen aufhalten oder wenigstens aufhalten können. Beispiele für einen solchen räumlichen Bereich sind öffentliche Plätze, Gebäude, Fahrzeuge, Lagereinrichtungen oder Industrieanlagen.

In diesem räumlichen Bereich kann mindestens ein Gas auftreten, welches bei einer ausreichend hohen Konzentration brennbar und / oder toxisch und / oder auf eine andere Weise für Menschen schädlich ist. Ein solches schädliches Gas wird nachfolgend als ein "Zielgas" bezeichnet.

Zwei stationäre Gasmessgeräte 1.1, 1.2 der Anordnung sind an zwei voneinander beabstandeten Orten aufgestellt. Die Bezeichnung "stationäres Gerät" spezifiziert, dass das Gerät dazu ausgestaltet ist, an einem Ort aufgestellt und / oder montiert und anschließend dort verwendet zu werden. Das stationäre Gerät vermag ein Signal zu generieren. Das Signal umfasst eine Information über ein Messergebnis des Gasmessgeräts 1.1, 1.2 und bevorzugt eine eindeutige Kennung des Gasmessgeräts 1.1, 1.2 und / oder einen Zeitstempel. Das stationäre Gerät 1.1, 1.2 umfasst eine Kommunikationseinheit, vermag ein Signal zu generieren und vermag mittels dieser Kommunikationseinheit das generierte Signal an einen räumlich entfernten Empfänger zu übermitteln. Die Kommunikationseinheit vermag das Signal insbesondere drahtgebunden und / oder per Radiowellen zu übermitteln. Das stationäre Gasmessgerät 1.1, 1.2 umfasst nicht notwendigerweise eine eigene Ausgabeeinheit, welche Messergebnisse oder Alarme in mindestens einer von einem Menschen wahrnehmbaren Form ausgibt. Optional umfasst das stationäre Gasmessgerät 1.1, 1.2 mindestens eine Statusleuchte, die einen internen Status des Geräts anzeigt.

Jedes Gasmessgerät 1.1, 1.2 weist jeweils einen schematisch gezeigten Detektionsbereich Det.1, Det.2 auf. Jedes Gasmessgerät 1.1, 1.2 vermag die Konzentration mindestens eines Zielgases in seinem Detektionsbereich Det.1, Det.2 zu messen. Die Detektionsbereiche Det.1, Det.2 decken zusammen wenigstens denjenigen Teil des räumlichen Bereichs ab, in dem sich Menschen aufhalten oder aufhalten können und in dem eine für Menschen schädliche Konzentration eines Zielgases auftreten kann.

Jedes Gasmessgerät 1.1, 1.2 vermag jeweils ein Signal zu generieren. Dieses Signal umfasst eine Informationen über die Zielgas-Konzentration, die dieses Gasmessgerät 1.1, 1.2 gemessen hat. In einer Ausgestaltung umfasst das Signal eine Information, ob die gemessene Zielgas-Konzentration oberhalb einer vorgegebenen oberen Schranke liegt oder nicht, aber nicht notwendigerweise die gemessene Zielgas-Konzentration selbst. Jede obere Schranke ist dergestalt vorgegeben, dass bei einer Zielgas-Konzentration kleiner als oder gleich der oberen Schranke keine Gefährdung für Menschen auftreten kann. In einer bevorzugten Ausgestaltung umfasst das Signal eine Kennzeichnung der gemessenen Zielgas-Konzentration. Ob dieses Zielgas-Konzentration zu hoch ist oder nicht, vermag ein nachfolgend beschriebenes Steuergerät 10 zu entscheiden. Diese Ausgestaltungen lassen sich miteinander kombinieren. Optional umfasst das Signal zusätzlich eine Information über die Geoposition oder eine eindeutige Kennung des Gasmessgeräts 1.1, 1.2 und / oder einen Zeitstempel für einen Messwert.

Möglich ist, dass die Gasmessgeräte 1.1, 1.2 alle das gleiche Zielgas zu detektieren vermögen. Möglich ist auch, dass die Gasmessgeräte 1.1, 1.2 insgesamt mindestens zwei verschiedene Zielgase zu detektieren vermögen.

Ein Rauchwarnmelder 1.3 vermag ein Indiz für das Auftreten von Rauch zu detektieren. Rauch ist bekanntlich häufig ein Indiz für ein Feuer. Auch der Rauchwarnmelder 1.3 weist einen Detektionsbereich Det.3 auf. Falls in diesem Detektionsbereich Det.3 Rauch auftritt, so detektiert der Rauchwarnmelder 1.3 diesen Rauch. Der Rauchwarnmelder 1.3 vermag diesem Fall ein Signal mit einer entsprechenden Information zu erzeugen.

Im gezeigten Ausführungsbeispiel werden zwei Gasmessgeräte 1.1, 1.2 und ein Rauchwarnmelder 1.3 verwendet. Selbstverständlich ist auch eine andere Anzahl von Gasmessgeräten und Rauchwarnmeldern möglich.

Bevorzugt umfassen jedes Gasmessgerät 1.1, 1.2 und der Rauchwarnmelder 1.3 jeweils eine Messkammer, und die Messkammer vermag eine Gasprobe aus dem jeweiligen Detektionsbereich Det.1, Det.2, Det.3 aufzunehmen. Unterschiedliche Detektionsprinzipien oder Messprinzipien sind möglich, wie ein Gasmessgerät 1.1, 1.2 ein Zielgas in einer Gasprobe zu detektieren vermag und / oder die Konzentration eines Zielgases in der Gasprobe zu messen vermag. Die beiden Gasmessgeräte 1.1, 1.2 können das gleiche Messprinzip oder unterschiedliche Messprinzipien anwenden. Beispielsweise emittiert eine Strahlungsquelle elektromagnetische Strahlung oder auch Schall in die Messkammer, ein Zielgas in der Messkammer schwächt die Strahlung oder den Schall ab, und ein Detektor misst die Intensität von auftreffender Strahlung oder auftreffendem Schall. Die gemessene Intensität korreliert mit der Zielgas-Konzentration. Auch der Rauchwarnmelder 1.3 kann ein entsprechendes Messprinzip anwenden, um Rauch in einer Gasprobe zu detektieren.

Ein signalverarbeitendes Steuergerät (control unit) 10 empfängt jeweils ein Signal der beiden Gasmessgeräte 1.1, 1.2 und des Rauchwarnmelders 1.3. Das Signal eines Gasmessgeräts 1.1, 1.2 enthält eine Information über die gemessene Zielgas-Konzentration, das Signal des Rauchwarnmelders 1.3 eine Information über die Detektion oder das Ausbleiben und optional der Intensität von Rauch. In einer Ausgestaltung enthält das Signal eines Gasmessgeräts 1.1, 1.2 die Information über eine gemessene Zielgas-Konzentration. Das Steuergerät 10 entscheidet, ob diese Zielgas-Konzentration oberhalb der vorgegebenen oberen Schranke ist. In einer anderen Ausgestaltung entscheidet das Gasmessgerät 1.1, 1.2 selbst, ob die Zielgas-Konzentration oberhalb der oberen Schranke liegt, und das Signal enthält eine Information über dieses Ergebnis.

Nachfolgend wird der Begriff "Alarmsituation" verwendet. Eine Alarmsituation ist aufgetreten, wenn eine Zielgas-Konzentration oberhalb der vorgegebenen oberen Schranke und / oder wenn Rauch detektiert worden sind. Eine Alarmsituation ist also auch dann aufgetreten, wenn der Rauchwarnmelder 1.3 Rauch detektiert hat.

In einer Ausgestaltung ermittelt das Steuergerät 10 einen zeitlichen Verlauf der Konzentration eines Zielgases in einem Detektionsbereich. Das Steuergerät 10 sagt abhängig vom zeitlichen Verlauf vorher, ob die Konzentration dieses Zielgases oberhalb der oberen Schranke liegen wird. Auch dieses Ereignis ist eine Alarmsituation.

Das Steuergerät 10 steuert eine Ausgabeeinheit 2 an. Diese Ausgabeeinheit 2 vermag abhängig von der Ansteuerung Meldungen auszugeben, die von einem Menschen wahrgenommen werden können, insbesondere akustisch. In einer Ausgestaltung erzeugt das Steuergerät 10 bei einer Alarmsituation automatisch aus vorgegebenen und abgespeicherten Sprachnachricht-Bausteinen eine Sprachmeldung. Die erzeugte Sprachmeldung kennzeichnet einerseits die aufgetretene Alarmsituation und andererseits Informationen über Maßnahmen, die jetzt zu treffen sind.

Die ausgegebene Sprachmeldung kennzeichnet die Alarmsituation beispielsweise durch folgende Informationen:
- Welches Zielgas hat die Alarmsituation ausgelöst? Oder hat die Detektion von Rauch die Alarmsituation ausgelöst?
- Wo sind dieses Zielgas oder der Rauch detektiert worden? Diese Information wird daraus abgeleitet, welches Gasmessgerät 1.1, 1.2 eine zu hohe Zielgas-Konzentration detektiert hat, und / oder, welcher Rauchwarnmelder 1.3 Rauch detektiert hat.
- optional eine Kennzeichnung der Zielgas-Konzentration oder der Stärke des Rauchs.

Zu den ausgegebenen Informationen über Maßnahmen, die jetzt zu treffen sind, können beispielsweise die folgenden Handlungen gehören:
- Der überwachte räumliche Bereich ist zu räumen.
- Die Lage eines Sammelpunkts, zu dem die Menschen im räumlichen Bereich sich begeben soll, wird beschrieben.
- Aufgelistet wird, ob und wenn ja welche persönliche Schutzausrüstung jetzt anzulegen ist.
- Optional wird beschrieben, wie persönliche Schutzausrüstung anzulegen ist.
- Optional wird beschrieben, welches Werkzeug jetzt eingesetzt werden soll.
- Der Weg zu einem Behälter mit Schutzausrüstung und Werkzeug wird beschrieben. Dieser Behälter wird weiter unten beschrieben.

In einer Ausgestaltung konfiguriert das Steuergerät 10 die Informationen über Maßnahmen abhängig davon, welches Gerät ein Signal mit einer Alarmsituation übermittelt hat. Falls beispielsweise ein Gasmessgerät 1.1, 1.2 ein toxisches Zielgas mit einer hohen Konzentration detektiert hat, so umfassen die Informationen über die Maßnahmen die Vorgabe, Gasmasken anzulegen, und optional die Vorgabe, ein tragbares Gasmessgerät mit sich zu führen. Falls ein Rauchwarnmelder 1.3 Rauch detektiert hat, so umfassen die Informationen beispielsweise die Vorgabe, einen Feuerlöscher oder eine Feuerlöschdecke zu verwenden.

Weiterhin steuert das Steuergerät 10 in einer Alarmsituation eine Anzeigeeinheit 3 an. Diese Anzeigeeinheit 3 zeigt in einer von einem Menschen wahrnehmbaren Form an, wohin man sich begeben soll, um einen nunmehr gefährlichen Bereich zu verlassen, insbesondere visuell. Im gezeigten Beispiel zeigt die Anzeigeeinheit 3 eine Richtung zu einem sicheren Bereich, beispielsweise zu einer Sammelstelle, in optischer Weise an. In einer Realisierungsform befindet sich die Anzeigeeinheit 3 in einem Ruhezustand, solange sie nicht angesteuert wird. Der Vorgang, dass das Steuergerät 10 die Anzeigeeinheit 3 ansteuert, bewirkt, dass die Anzeigeeinheit 3 in einen Anzeigezustand überführt wird. In einer Realisierungsform bewirkt die Ansteuerung, dass die Anzeigeeinheit 3 beleuchtet wird und / oder blinkt.

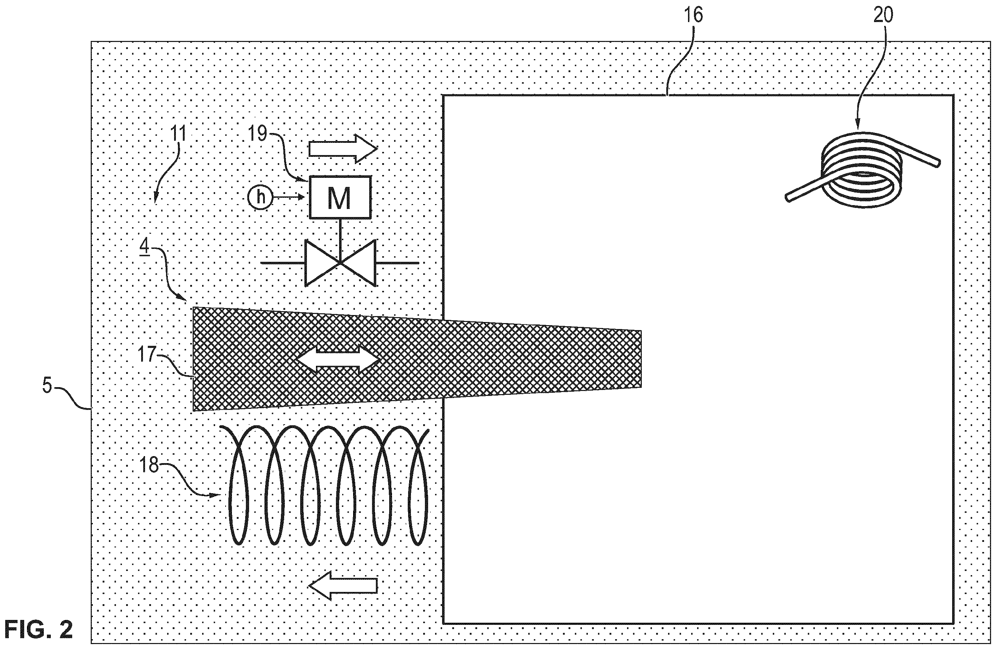

Nachfolgend wird der bereits erwähnte Behälter mit Schutzausrüstung und Werkzeug näher beschrieben. Der verschließbarer Behälter 5 umfasst eine Wand 11, die einen Innenraum In umschließt. Der Behälter 5 ist beispielsweise als ein Schrank ausgestaltet. In die Wand 11 sind eine Öffnung und ein Verschluss in Form einer Tür 16 eingelassen, vgl. Figur 2. Die Tür 16 lässt sich relativ zur Wand 11 um eine vertikale Drehachse drehen und dadurch öffnen und schließen. Die vertikale Drehachse liegt in der Zeichenebene von Figur 1 und der von Figur 2. Die geschlossene Tür verschließt die Öffnung in der Wand 11. Außen an der Wand 11 sind im Ausführungsbeispiel eine akustische Meldeeinheit 12, beispielsweise eine Hupe, sowie eine optische Meldeeinheit 13, beispielsweise eine blinkende Meldeleuchte 13, angeordnet.

Der Innenraum In ist dazu ausgestaltet, mehrere Gegenstände aufzunehmen. Im Innenraum In können sich mehrere Regalfächer befinden. Diese Gegenstände lassen sich dafür verwenden, um aus einem Bereich mit einer hohen Zielgas-Konzentration zu entkommen, und / oder um das Austreten von Zielgas oder die Ausbreitung eines Feuers zu bekämpfen. In Figur 1 werden beispielhaft folgende Gegenstände gezeigt, die sich aktuell im Innenraum In befinden:
- eine Fluchthaube 6, die ein Benutzer sich vor das Gesicht anlegen kann und die einen Filter für Partikel umfasst,
- einen Selbstretter 7 in einem eigenen Behälter, wobei der Selbstretter 7 eine Gesichtsmaske und einen Sauerstoffgenerator aufweist und dazu ausgestaltet ist, ein Gasgemisch umfassend Sauerstoff zu generieren und zur Gesichtsmaske zu leiten,
- ein tragbares Gasmessgerät 8, welches ein Mensch mit sich führen kann,
- einen Feuerlöscher 9 und
- eine Taschenlampe 14.

Weitere Gegenstände, die sich im Behälter 5 befinden können, sind beispielsweise
- ein Schutzhelm,
- eine Gasmaske mit einer Filtereinheit,
- eine Leiter,
- Werkzeug wie Beil und oder Hammer oder Ramme und
- ein akustisches Warngerät, beispielsweise eine tragbare Hupe oder Sirene.

Ein Verschließmechanismus 4 vermag wahlweise die Tür 16 und damit den Behälter 5 zu verschließen oder einen Zugriff auf die Gegenstände 6, 7, 8, 9, 14 im Behälter 5 zu ermöglichen. Wenn der Verschließmechanismus 4 in einem verschließenden Zustand ist, ist die Tür 16 verschlossen und arretiert, und ein Zugriff auf die Gegenstände 6, 7, 8, 9, 14 im Behälter 5 wird unterbunden. Der Verschließmechanismus 4 ist also wahlweise im verschließenden oder in einem freigebenden Zustand. Im Ausführungsbeispiel umfasst der Behälter 5 die Tür 16. Im verschließenden Zustand verschließt und arretiert der Verschließmechanismus 4 diese Tür 16. Wenn der Verschließmechanismus 4 im freigebenden Zustand ist, lässt die Tür 16 sich öffnen oder wird automatisch geöffnet.

Figur 2 zeigt eine Ausgestaltung des Verschließmechanismus 4. In dieser Ausgestaltung lässt sich ein Arretierkörper 17 des Verschließmechanismus 4 relativ zur Wand 11 und damit zur Tür 16 zwischen einer verschließenden Position und einer freigebenden Position hin und her bewegen, im Ausführungsbeispiel waagerecht hin und her bewegen. In der verschließenden Position verschließt und arretiert der Arretierkörper 17 die Tür 16. Der Verschließmechanismus 4 ist dann im verschließenden Zustand. Figur 2 zeigt diese verschließende Position. In der freigebenden Position ermöglicht der Arretierkörper 17, dass die Tür 16 geöffnet wird. Der Verschließmechanismus 4 ist dann im freigebenden Zustand.

In der gezeigten Realisierungsform umfasst der Verschließmechanismus 4 weiterhin ein passives Rückstellelement, beispielsweise eine mechanische oder pneumatische Feder, die sich an der Wand 11 abstützt, sowie ein Stellglied 19. Die Bezeichnung "passiv" spezifiziert, dass das Rückstellelement 18 keine Versorgung mit elektrischer, pneumatischer und / oder hydraulischer Energie und auch keine Ansteuerung von außen benötigt. Das Rückstellelement 18 übt dauerhaft eine rückstellende Kraft aus, im Beispiel von Figur 2 nach links. Das Rückstellelement 18 ist bestrebt, den Arretierkörper 17 in die eine Position zu bewegen und in dieser Position zu halten. Durch eine entsprechende Ansteuerung von außen lässt sich das Stellglied 19 aktivieren und deaktivieren. Das Steuergerät 10 vermag das Stellglied 19 anzusteuern. Das aktivierte Stellglied 19 ist bestrebt, den Arretierkörper 17 gegen die rückstellende Kraft des Rückstellelement 18 in die andere Position zu bewegen, im Beispiel von Figur 2 nach rechts. Falls das Stellglied 19 deaktiviert ist, so wirkt nur das Rückstellelement 18 auf den Arretierkörper 17.

In einer Realisierungsform ist das Rückstellelement 18 bestrebt, den Arretierkörper 17 in die verschließende Position zu bewegen. Das aktivierte Stellglied 19 verschiebt den Arretierkörper 17 in die freigebende Position. In Figur 2 wird die entgegengesetzte Realisierungsform beschrieben. Das Rückstellelement 18 umfasst in diesem Beispiel eine Zugfeder und ist bestrebt, den Arretierkörper 17 in die freigebende Position zu bewegen, hier also nach links zu ziehen. Das aktivierte Stellglied 19 ist bestrebt, den Arretierkörper 17 in die verschließende Position zu bewegen, hier also nach rechts zu schieben.

In einer Ausgestaltung ist ein passives Öffnungselement, beispielsweise eine Drehfeder 20, bestrebt, die Tür 16 zu öffnen, so dass die Tür 16 aufspringt, und im geöffneten Zustand zu halten. Diese Ausgestaltung bewirkt folgendes: Sobald der Verschließmechanismus 4 in den freigebenden Zustand überführt wird und die Tür 16 nicht mehr arretiert ist, öffnet das Öffnungselement 20 die Tür 16, ohne dass hierfür eine Handlung eines Menschen erforderlich ist. Die Tür 16 springt also von allein auf.

Der Verschließmechanismus 4 lässt sich von außen ansteuern. Bevorzugt befindet der Verschließmechanismus 4 sich im verschließenden Zustand, solange er nicht angesteuert wird. Eine Ansteuerung bewirkt, dass der Verschließmechanismus 4 in den freigebenden Zustand überführt wird. Im Beispiel von Figur 2 bewirkt eine Ansteuerung des Stellglieds 19, dass das Stellglied 19 deaktiviert wird und dadurch das Rückstellelement 18 den Arretierkörper 17 in die freigebende Position bewegt.

Figur 2 zeigt die folgende Realisierungsform: Solange keine unzulässige Zielgas-Konzentration und keine sonstige Alarmsituation detektiert wird, ist das Stellglied 19 aktiviert und hält den Arretierkörper 17 in der verschließenden Position. Falls das Steuergerät 10 eine unzulässige Zielgas-Konzentration oder eine sonstige Alarmsituation detektiert hat und daher ein Zugriff auf Gegenstände im Behälter 4 notwendig ist, steuert das Steuergerät 10 das Stellglied 19 an. Als Reaktion hierauf wird das Stellglied 19 deaktiviert, und das Rückstellelement 18 bewegt den Arretierkörper 17 in die freigebende Position.

Wie bereits dargelegt, detektiert das Steuergerät 10 eine Alarmsituation insbesondere dann, wenn eines der folgenden Ereignisse aufgetreten ist:
- Ein Gasmessgerät 1.1, 1.2 hat eine Zielgas-Konzentration oberhalb der oberen Schranke gemessen.
- Der Rauchwarnmelder 1.3 hat Rauch detektiert.

Falls das Steuergerät 10 eine Alarmsituation detektiert hat, so löst das Steuergerät 10 die folgenden Schritte aus:
- Das Steuergerät 10 steuert den Verschließmechanismus 4 an und bewirkt dadurch, dass der Verschließmechanismus 4 in den freigebenden Zustand überführt wird, in einer Realisierungsform vom Rückstellelement 18 und in einer anderen Realisierungsform gegen die rückstellende Kraft des Rückstellelements 18.
- Die Tür 16 springt auf oder wird von Hand geöffnet. Anschließend lassen sich die Gegenstände 6, 7, 8, 9, 14 aus dem Behälter 5 entnehmen.
- Das Steuergerät 10 steuert die akustische Meldeeinheit 12 an und bewirkt, dass diese einen Ton ausgibt.
- Das Steuergerät 10 steuert die optische Meldeeinheit 13 an und bewirkt, dass diese eine visuell wahrnehmbare Meldung ausgibt.

Die angesteuerten Meldeeinheiten 12 und 13 erleichtern es einem Benutzer, den Schrank 5 zu finden, auch in einer Alarmsituation.

### Bezugszeichenliste

| | |
|---|---|
| 1.1, 1.2 | stationäres Gasmessgerät, misst in seinem jeweiligen Detektionsbereich Det.1, Det.2 die Konzentration eines schädlichen Zielgases |
| 1.3 | Rauchwarnmelder, hat den Detektionsbereich Det.3 |
| 2 | akustische Ausgabeeinheit |
| 3 | optische Fluchtweg-Anzeige |
| 4 | ansteuerbarer Verriegelungsmechanismus für den Schrank 5, umfasst den Arretierkörper 17, das Rückstellelement 18 und das Stellglied 19, vermag die Tür 16 wahlweise zu verriegeln oder freizugeben |
| 5 | Schrank, umfasst die Wand 11 und die Tür 16 in der Wand 11, nimmt in seinem Innenraum die Fluchthaube 6, den Selbstretter 7, das Gasmessgerät 8, den Feuerlöscher 9 und die Taschenlampe 14 auf, vom Verriegelungsmechanismus 4 verschließbar, fungiert als der Behälter |
| 6 | Fluchthaube im Schrank 5, umfasst einen Partikelfilter und eine Gesichtsmaske |
| 7 | Selbstretter im Schrank 5, umfasst einen Sauerstoffgenerator und eine Fluchthaube in einem Behälter |
| 8 | tragbares Gasmessgerät im Schrank 5 |
| 9 | Feuerlöscher im Schrank 5 |
| 10 | signalverarbeitendes Steuergerät, empfängt jeweils ein Signal von jedem stationären Gasmessgerät 1.1, 1.2 und vom Rauchwarnmelder 1.3, steuert die Lautsprecher 2, die Fluchtweg-Anzeige 3, den Verriegelungsmechanismus 4 und die Meldeeinheiten 12, 13 an |
| 11 | Wand des Behälters 5, trägt die Tür 16 sowie die Einheiten 12 und 13 |
| 12 | akustische Meldeeinheit |
| 13 | visuelle Meldeeinheit |
| 14 | Taschenlampe im Schrank 5 |
| 16 | Tür in der Wand 11, um eine vertikale Achse drehbar |
| 17 | Arretierkörper, zwischen einer verschließenden und einer freigebenden Position hin und her beweglich |
| 18 | Rückstellelement in Form einer Zugfeder, ist bestrebt, den Arretierkörper 17 in die freigebende Position zu bewegen |
| 19 | ansteuerbares Stellglied, ist bestrebt, den Arretierkörper 17 in die verschließende Position zu bewegen |
| 20 | Öffnungsmechanismus für die Tür 16, umfasst eine Drehfeder |
| Det.1, Det.2 | Detektionsbereich des Gasmessgeräts 1.1, 1.2 |
| Det.3 | Detektionsbereich des Rauchwarnmelders 1.3 |
| In | Innenraum des Behälters 5, von der Wand 11 umschlossen |

## Patentansprüche

1. Anordnung umfassend
- mindestens ein Gasmessgerät (1.1, 1.2), wobei das oder jedes Gasmessgerät (1.1, 1.2) der Anordnung jeweils einen Detektionsbereich (Det.1, Det.2) aufweist,
- einen Behälter (5) mit einer Wand (11), die einen Innenraum (In) umgibt,
- einen Verschließmechanismus (4) für den Behälter (5) und
- ein signalverarbeitendes Steuergerät (10),
wobei das oder jedes Gasmessgerät (1.1, 1.2) dazu ausgestaltet ist,
- an jeweils einem Ort aufgestellt zu werden,
- in seinem Detektionsbereich (Det.1, Det.2) die jeweilige Konzentration mindestens eines Zielgases zu messen und
- ein Signal zu generieren, welches eine Information über die gemessene Zielgas-Konzentration umfasst,
wobei der Behälter (5) dazu ausgestaltet ist, in seinem Innenraum (In) mindestens einen Gegenstand (6, 7, 8, 9, 14) aufzunehmen,
wobei der Verschließmechanismus (4) wahlweise in einen verschließenden und in einen freigebenden Zustand überführbar ist,
wobei der Verschließmechanismus (4)
- im verschließenden Zustand den Behälter (5) derart verschließt, dass ein Zugriff auf einen im Behälter (5) befindlichen Gegenstand (6, 7, 8, 9, 14) versperrt ist, und
- im freigebenden Zustand eine Entnahme eines Gegenstands (6, 7, 8, 9, 14) aus dem Behälter (5) ermöglicht,
wobei das Steuergerät (10) dazu ausgestaltet ist,
- von dem oder jedem Gasmessgerät (1.1, 1.2) der Anordnung das jeweilige Signal zu empfangen und
- abhängig von dem oder jedem empfangenen Signal zu entscheiden, ob in mindestens einem Detektionsbereich (Det.1, Det.2) eines Gasmessgeräts (1.1, 1.2) der Anordnung mindestens ein Zielgas mit einer Konzentration außerhalb eines für dieses Zielgas vorgegebenen Wertebereichs, insbesondere oberhalb einer vorgegebenen oberen Schranke, auftritt, und
wobei das Steuergerät (10) weiterhin dazu ausgestaltet ist,
als Reaktion darauf, dass in dem oder mindestens einem Detektionsbereich (Det.1, Det.2) mindestens ein Zielgas mit einer Konzentration außerhalb des Wertebereichs auftritt,
zu bewirken, dass der Verschließmechanismus (4) in den freigebenden Zustand überführt wird.

2. Anordnung nach Anspruch 1,
**dadurch gekennzeichnet, dass**
die Anordnung weiterhin einen Rauchwarnmelder (1.3) umfasst, der einen Detektionsbereich (Det.3) aufweist,
wobei der Rauchwarnmelder (1.3)
- an einem Ort angeordnet ist und
- dazu ausgestaltet ist,
das Ereignis zu detektieren, dass in seinem Detektionsbereich (Det.3) Rauch vorhanden ist, und
ein Signal umfassend eine Information über das Vorhandensein von Rauch zu generieren, und
wobei das Steuergerät (10) dazu ausgestaltet ist,
- das Signal des Rauchwarnmelders (1.3) zu empfangen und
- als Reaktion auf den Empfang dieses Signals zu bewirken,
dass der Verschließmechanismus (4) in den freigebenden Zustand überführt wird.

3. Anordnung nach einem der vorhergehenden Ansprüche,
**dadurch gekennzeichnet, dass**
die Anordnung mindestens eine Meldeeinheit (12, 13) umfasst,
wobei die Meldeeinheit (12, 13)
- am Behälter (5) angeordnet ist oder
- dazu ausgestaltet ist, von einem Menschen getragen zu werden,
wobei die oder jede Meldeeinheit (12, 13) der Anordnung dazu ausgestaltet ist,
- aktiviert zu werden und
- als Reaktion auf eine Aktivierung eine von einem Menschen von außerhalb des Behälters (5) wahrnehmbare Ausgabe zu tätigen, und
wobei das Steuergerät (10) dazu ausgestaltet ist,
als Reaktion auf die Detektion des Ereignisses, dass die oder mindestens eine Zielgas-Konzentration außerhalb des jeweiligen Wertebereichs liegt,
die oder mindestens eine Meldeeinheit (12, 13) anzusteuern und dadurch zu aktivieren.

4. Anordnung nach einem der vorhergehenden Ansprüche,
**dadurch gekennzeichnet, dass**
die Anordnung mindestens einen der folgenden Gegenstände umfasst,
wobei diese Gegenstände sich im Innenraum (In) des Behälters (5) befinden:
- ein Bestandteil (6, 7) einer persönlichen Schutzausrüstung,
- eine Quelle für Atemluft,
- ein Werkzeug (9) zum Bekämpfen eines Brandes,
- ein Werkzeug zum Herstellen oder Vergrößern eines Zugangs zu einem Raum,
- ein Werkzeug zum Abdichten eines Lecks,
- eine tragbare Lichtquelle (14),
- ein tragbares Gasmessgerät (8).

5. Anordnung nach einem der vorhergehenden Ansprüche,
**dadurch gekennzeichnet, dass**
die Anordnung zusätzlich eine ansteuerbare Anzeigeeinheit (3) umfasst,
wobei die Anzeigeeinheit (3) dazu ausgestaltet ist, als Reaktion auf eine Ansteuerung mindestens eine Information in mindestens einer von einem Menschen außerhalb des Behälters (5) wahrnehmbaren Art auszugeben,
wobei die oder eine Information eine Warnung und / oder eine Richtungsanzeige und / oder eine Kennzeichnung einer räumlichen Position ist, und
wobei das Steuergerät (10) dazu ausgestaltet ist, dann, wenn in mindestens einem Detektionsbereich (Det.1, Det.2) eines Gasmessgeräts (1.1, 1.2) der Anordnung mindestens ein Zielgas mit einer Konzentration außerhalb des Wertebereichs auftritt,
optional auch dann, wenn in Detektionsbereich (Det.3) des Rauchwarnmelders (1.3) Rauch vorhanden ist,
die Anzeigeeinheit (3) anzusteuern und dadurch die Ausgabe einer Information zu bewirken.

6. Anordnung nach einem der vorhergehenden Ansprüche,
**dadurch gekennzeichnet, dass**
der Behälter einen Verschluss (16), insbesondere eine Tür, umfasst und
der Verschließmechanismus (4)
- einen Arretierkörper (17),
- ein Rückstellelement (18) und
- ein aktivierbares und deaktivierbares Stellglied (19)
umfasst,
wobei der Verschluss (16) in die Wand (11) des Behälters (5) eingelassen ist und geöffnet und geschlossen werden kann,
wobei der geöffnete Verschluss (16) die Entnahme eines Gegenstands (6, 7, 8, 9, 14) aus dem Behälter (5) ermöglicht,
wobei der Arretierkörper (17) relativ zum Verschluss (16) zwischen
- einer verschließenden Position, in der der Arretierkörper (17) den Verschluss (16) verschließt, und
- einer freigebenden Position, in der der Verschluss (16) geöffnet werden kann,
hin und her beweglich ist,
wobei das Rückstellelement (18) bestrebt ist,
- eine rückstellende Kraft auszuüben und
- dadurch den Arretierkörper (17) in die eine der beiden Positionen zu bewegen,
wobei das aktivierte Stellglied (19) dazu ausgestaltet ist, den Arretierkörper (17) gegen die rückstellende Kraft in die andere der beiden Positionen zu bewegen, und
wobei das Steuergerät (10) dazu ausgestaltet ist, das Stellglied (19) anzusteuern und durch die Ansteuerung zu aktivieren oder zu deaktivieren.

7. Anordnung nach Anspruch 6,
**dadurch gekennzeichnet, dass**
die vom Rückstellelement (18) ausgeübte rückstellende Kraft bestrebt ist, den Arretierkörper (17) in die freigebende Position zu bewegen, und
das aktivierte Stellglied (19) dazu ausgestaltet ist, den Arretierkörper (17) gegen die rückstellende Kraft in die verschließende Position zu bewegen,
wobei das Steuergerät (10) dazu ausgestaltet ist,
dann, wenn in dem oder mindestens einem Detektionsbereich (Det.1, Det.2) mindestens ein Zielgas mit einer Konzentration außerhalb des Wertebereichs auftritt,
- das Stellglied (19) anzusteuern und dadurch zu deaktivieren und
- durch diese Ansteuerung zu bewirken, dass der Arretierkörper (17) durch die rückstellende Kraft in die freigebende Position bewegt wird.

8. Verfahren zum Überwachen eines räumlichen Bereichs,
wobei das Verfahren unter Verwendung einer Anordnung durchgeführt wird,
wobei die verwendete Anordnung
- mindestens ein Gasmessgerät (1.1, 1.2),
- einen Behälter (5) mit einer Wand (11), die einen Innenraum (In) umgibt,
- einen Verschließmechanismus (4) für den Behälter (5) und
- ein signalverarbeitendes Steuergerät (10),
umfasst,
wobei der Innenraum (In) des Behälters (5) mindestens einen Gegenstand (6, 7, 8, 9, 14) aufnimmt,
wobei der Verschließmechanismus (4)
- sich anfangs in einem verschließenden Zustand befindet und
- den Behälter (5) derart verschließt, dass ein Zugriff auf einen im Behälter (5) befindlichen Gegenstand (6, 7, 8, 9, 14) versperrt ist,
wobei das oder jedes Gasmessgerät (1.1, 1.2) der Anordnung
- an jeweils einem Ort aufgestellt ist und
- jeweils einen Detektionsbereich (Det.1, Det.2) aufweist,
wobei das Verfahren die automatisch durchgeführten Schritte umfasst, dass
das oder jedes Gasmessgerät (1.1, 1.2)
- wiederholt in seinem Detektionsbereich (Det.1, Det.2) die jeweilige Konzentration mindestens eines Zielgases misst und
- ein Signal generiert, welches eine Information über die gemessene Zielgas-Konzentration umfasst, und
das Steuergerät (10)
- von dem oder jedem Gasmessgerät (1.1, 1.2) der Anordnung das jeweilige Signal empfängt und
- abhängig von dem oder jedem empfangenen Signal automatisch entscheidet, ob im jeweiligen Detektionsbereich (Det.1, Det.2) mindestens eines Gasmessgeräts (1.1, 1.2) der Anordnung mindestens ein Zielgas mit einer Konzentration außerhalb eines für dieses Zielgas vorgegebenen Wertebereichs, insbesondere oberhalb einer vorgegebenen oberen Schranke, auftritt, und
wobei das Verfahren den weiteren automatisch durchgeführten Schritt umfasst, dass das Steuergerät (10)
als Reaktion darauf, dass in dem oder mindestens einem Detektionsbereich (Det.1, Det.2) mindestens ein Zielgas mit einer Konzentration außerhalb des Wertebereichs auftritt,
bewirkt, dass der Verschließmechanismus (4) in einen freigebenden Zustand überführt wird,
wobei der Verschließmechanismus (4) im freigebenden Zustand eine Entnahme eines Gegenstands (6, 7, 8, 9, 14) aus dem Behälter (5) ermöglicht.
